# EUROPEAN PATENT APPLICATION

(11) **EP 3 888 545 A1**
(43) Date of publication of application: **06.10.2021**
(21) Application number: 20167502.2
(22) Date of filing: 01.04.2020
(51) Int. Cl.: A61B 5/06, A61B 5/00, A61B 5/055

(54) **A DEVICE FOR DETERMINING A POSITION OF A METAL OBJECT IN OR AT A PATIENT BODY**

(71) Applicant: Koninklijke Philips N.V., 5656 AG Eindhoven (NL)
(72) Inventor: CHAUDHURY, Sudipta, 5656 AE Eindhoven (NL); SISODIA, Rajendra Singh, 5656 AE Eindhoven (NL); WEISS, Steffen, 5656 AE Eindhoven (NL); VOGTMEIER, Gereon, 5656 AE Eindhoven (NL)
(74) Representative: Philips Intellectual Property & Standards

(57) **Abstract**

The invention is directed to a device (100) for determining a position of a metal object (200) in or at a patient body (300), the device (100) comprising:
-a sensor unit (102) configured to be moved along a pre-determined trajectory (104),
wherein the sensor unit (102) is configured for generating a magnetic field (106) during a movement of the sensor unit (102) along the trajectory (104),
wherein the sensor unit (102) is configured for receiving a response signal induced by the generated magnetic field (106),
wherein the sensor unit (102) is configured for detecting a change of the response signal induced by the generated magnetic field (106) during said movement due to a change (110) of a distance between the sensor unit (102) and the metal object (200) in or at the patient body (300), and
wherein in the sensor unit (102) is configured for determining the position of the metal object (200) based on the detected change (110) of the response signal during said movement.

## Description

### FIELD OF THE INVENTION

The invention is directed to a device for determining a position of a metal object in or at a patient body, a method for determining a position of a metal objection in or at a patient body, a method for autonomous imaging, and a computer program element.

### BACKGROUND OF THE INVENTION

Patient safety is paramount during any imaging. In an autonomous imaging system, suitability of a patient for scanning and subsequent preparation is required. The patient needs to be prevented from injury even more precisely as there may or may not be any human intervention possible towards any unwanted situations.

Presence of metal objects during MRI scan can harm patient due to presence of a powerful magnetic field. Metal detectors either in the form of a door, such as an airport or handheld metal detectors are used to detect presence of metal objects. Furthermore, safety questions are asked to the patients related to any metal objects, implants, and/or jewelry which a patient may wear.

### SUMMARY OF THE INVENTION

With embodiments of the invention, an improved device is provided for determining a position of a metal object in or at a patient body.

The present invention is defined by the independent claims. Further embodiments and advantages of the present invention are incorporated in the dependent claims and the description.

Technical terms are used in a common sense. If a specific meaning is conveyed to certain terms, a definition of terms will be given in the following in which the terms are used.

According to a first aspect of the invention, a device for determining a position of a metal object in or at a patient body comprises a sensor unit configured to be moved along a predetermined trajectory. Furthermore, the sensor unit is configured for generating a magnetic field during a movement of the sensor unit along the trajectory. In addition, the sensor unit is configured for receiving a response signal induced by the generated magnetic field. In addition, the sensor unit is configured for detecting a change of the response signal induced by the generated magnetic field during said movement due to a change of a distance between the sensor unit and a metal object in or at the patient body. Furthermore, the sensor unit is configured for determining the position of the metal object based on the detected change of the response signal during said movement.

The advantage of this aspect may be that with the help of the sensor unit and the device, a presence and a position of a metal object can be detected. This is beneficial for MRI and X-ray examinations where the presence of a metal object is critical, since the object may be pulled towards the magnetic field or generates artefacts in the resulting medical image. In addition, the labor can be reduced for preparing a patient for a medical imaging procedure, since the device may enable the patient to remove the metal object by himself, since the position of the metal object may be indicated by the device. Furthermore, the device may have the advantage that operating personnel of a medical imaging modality can be unburdened, since the device detects the metal object and the medical personnel only needs to double-check whether all metal objects have been removed or not. Furthermore, because precise location of the metal object in and/or at body parts may be known, an automated instructions can be generated to guide users to remove said metal object.

In other words, the device is configured for determining, calculating and/or estimating a position, respectively a location, of a metal object, e.g. a magnetic object, in or at a patient body, respectively, adjacent to a patient body. Furthermore, the device comprises and/or consists of at least a sensor unit, respectively, a sensor device configured to be moved along and/or shifted along a predetermined, respectively, a planned trajectory and/or path. Furthermore, the sensor unit is configured for generating, creating and/or establishing a magnetic field, respectively, a magnetic induction during and/or for the time of a movement, respectively, motion of the sensor unit along the trajectory. In addition, the sensor unit is configured for receiving, detecting and/or identifying a response signal, respectively, a reaction induced and/or caused by the generated magnetic field. Furthermore, the sensor unit is configured for detecting, identifying and/or measuring a change, respectively, and alteration of the response signal induced by the generated magnetic field during said movement due to a change, a difference and/or a variation of a distance, vector and/or range between the sensor unit and a metal object in the or at the patient body. Moreover, the sensor unit is configured for determining and/or calculating the position of the metal object based on the detected change of the response signal during said movement. The known position of the metal object and/or the device position may be used to give context- and location-aware instructions to the user or also staff to remove the metal objects, e.g. via natural language generation techniques to generate these instructions.

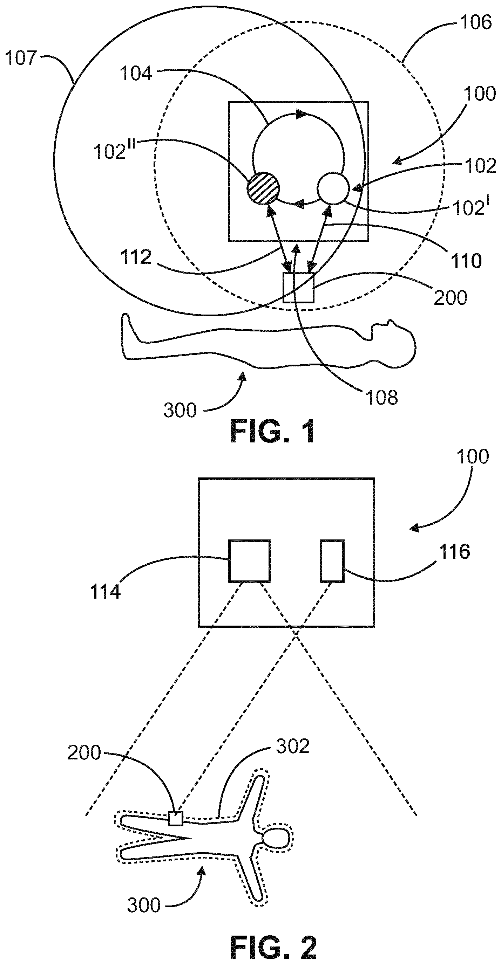

For example, the sensor unit comprises a device which is able to generate a magnetic field and also able to detect a change of the magnetic field and thereby generates a signal which is indicative for the change of the magnetic field due to the presence of a metal object. Said response signal varies due to the movement of said device and/or sensor unit along a predetermined trajectory and based on said change, the position of the metal object can be calculated. This is explained in full detail in view of Figure 1 and the corresponding Figure description.

According to an embodiment of the invention, the change of the response signal is dependent from the trajectory.

This embodiment may have the advantage that due to the correlation between the change of the response signal and the trajectory, the position of the metal object can be calculated more easily and more precisely.

The change of the response signal is dependent from, correlates to and/or is linked to the trajectory. For the movement of the sensor unit along the predetermined trajectory, it correlates to the change of the response signal and thereby the position of the metal object can be determined.

According to an embodiment, the device comprises a camera. Furthermore, the camera is configured for generating an image of the patient body. The camera is configured for identifying a contour of the patient body based on the generated image. Moreover, the device is configured for adapting a trajectory based on the identified contour of the patient body.

This embodiment may have the advantage that metal objects are detected more easily and more precisely, since the contour of the patient body is considered in the predetermined trajectory.

In other words, the device comprises and/or has a camera, an imaging device and/or an image sensor. The camera may be configured for generating and/or acquiring an image, respectively a picture, of the patient body. Moreover, the camera is configured for identifying, rendering and/or calculating a contour, a border and/or a boundary of the patient body based on the generated image. Moreover, the device is configured for adapting, changing and/or altering the trajectory based on the identified contour of the patient body. This embodiment is explained in full detail in view of Figure 2 and the corresponding Figure description.

According to an embodiment, the device comprises an optical indicator, e.g. a laser beam. Furthermore, the optical indicator is configured for illuminating the position of the metal object based on the determined position of the metal object.

This embodiment may have the advantage that the metal object can be more easily identified and removed, since the optical indicator identifies the position of the metal object by illuminating the metal object on the patient body.

In other words, the device comprises and/or has an optical indicator, respectively, a device for generating an optical indicator, e.g. a laser beam. Furthermore, the optical indicator is configured for illuminating, highlighting and/or presenting the position of the metal object based on the determined position of the metal object. For example, the patient has a metal object in his trouser pocket. With the help of the device, the position of the metal object, in particular the position of the metal object in the trouser can be determined. With the help of the optical indicator, a laser beam is directed to the pocket of the trouser and thereby, the laser beam highlights the pocket of the trouser and, therefore, the metal object can be removed. This guidance may be altered by audio information (e.g. natural language generation) and further video guidance.

According to an embodiment, the sensor unit is located inside of a wall element, a chair and/or an examination table.

This embodiment may have the advantage that the device increases the usability for a patient, since the patient only has to stand in front of the wall element and metal objects can be located.

In other words, the sensor unit is located inside, is part of and/or is formed in one piece of a wall element, a chair and/or an examination table. For example, the sensor unit is located movably inside the wall element. The patient has to stand in front of the wall and the sensor unit moves along the predetermined trajectory inside the wall element such that the position of the metal object can be determined.

According to an embodiment, the sensor unit comprises a first sub-sensor unit which moves along the trajectory. Furthermore, the sensor unit comprises a second sub-sensor unit, and a third sub-sensor unit which both are located at a fixed position. In addition, the device is configured for determining the position of the metal object based on the detected change of the distance during a movement of the first sub-sensor unit along a predefined trajectory and the fixed positions of the second sub-sensor unit and a third sub-sensor unit.

This embodiment may have the advantage that with the help of the two sub-sensor units having a fixed position, the detection of a metal object can be double-checked, since the first sub-sensor unit still moves along the trajectory and, therefore, can detect the position of the metal object. Therefore, a two-level security check for metal objects can be achieved.

In other words, the sensor unit comprises a first sub-sensor unit, respectively, a movable first sub-sensor unit, which moves along the trajectory. In addition, the sensor unit comprises a second sub-sensor unit and a third sub-sensor unit which both are located at a fixed position, respectively, are not movable. Moreover, the device is configured for determining, calculating and/or detecting the position of the metal object based on the detected change of the distance during a movement, motion and/or shift of the first sub-sensor unit along the predefined trajectory and the fixed positions of the second sub-sensor unit and a third sub-sensor unit. For example, the second and third sub-sensor unit, both having fixed positions, may detect the presence of a metal object in or at the patient body but are not able to locate the position of the metal object. In the case that the presence of a metal object is detected, the first sub-sensor unit starts moving along the trajectory and determines the location and/or position of the metal object based on the detected change of the distance during said movement.

According to an embodiment, the sensor unit comprises a plurality of sub-sensor units. Each sub-sensor unit of the plurality of sub-sensor units is movably arranged inside a wall. In addition, the device is configured for calculating a shift for each of the sub-sensor units for relocating each of the sub-sensor units of the plurality of sub-sensor units based on a pattern. Moreover, the device is configured for moving each sub-sensor unit of the plurality of sub-sensor units based on the calculated shift.

This embodiment may have the advantage that with the help of the plurality of sub-sensors, a metal object can be detected much quicker, since each sub-sensor unit is able to detect the metal object.

In other words, the sensor unit comprises, respectively, has a plurality, respectively, a high number of sub-sensor units. Each sub-sensor unit of the plurality of sub-sensor units is movably arranged, can be moved and/or can be shifted inside a wall, respectively, a wall element. Furthermore, the device is configured for calculating, detecting and/or estimating a shift, respectively, a movement or a motion for each of the sub-sensor units for relocating and/or repositioning each of the sub-sensor units of the plurality of sub-sensor units based on a pattern, respectively, a cluster. Moreover, the device is configured for moving and/or shifting each sub-sensor unit of the plurality of the sub-sensor units based on the calculated shift.

According to an embodiment, the device is configured for adapting the pattern based on a received probability value, describing where to find metal objects in or at the patient body.

The advantage of this embodiment can be that the pattern is adapted towards regions which have a high chance of having metal objects, for example, pockets and/or the neck region for jewelry.

In other words, the device is configured for adapting and/or changing the pattern based on a received probability value or a likelihood, describing and/or indicating where to find metal objects in or at the patient body. The probability value is understood in this context as a likelihood or a chance to detect a metal object in a specific region, e.g. a neck region.

According to an embodiment, the device is configured for changing a sensitivity of each of the sub-sensor units of the plurality of sub-sensor units based on the pattern and the probability.

This embodiment may have the advantage that the presence of metal objects can be detected more reliably, since the sensitivity can be individually adapted of the sub-sensor units.

In other words, the device is configured for changing, respectively, adapting a sensitivity and/or a measuring rate of each of the sub-sensor units of the plurality of sub-sensor units based on the pattern and the probability.

According to an embodiment, the device is configured for adapting the pattern based on the identified contour of the patient body.

This embodiment may have the advantage that with the identified contour of the patient body, the regions where a probability to detect metal objects is very high, can be used for adapting the pattern and, therefore, the reliability of the device can be further improved.

In other words, the device is configured for adapting and/or changing the pattern based on the identified contour of the patient body. Moreover, in an example, the pattern is adapted by identifying a high probability of the metal objects in a specific region, e.g. a neck region of the patient, based on the image and the contour of the patient body.

According to an embodiment, each sub-sensor unit comprises a generating element and a detecting element. Each of the generating elements is configured for generating a continuously alternating magnetic field at a predefined frequency. In addition, each of the generating elements is paired with one detecting element. Furthermore, the generating elements operate at a different predefined frequency. Moreover, each of the detecting elements is configured for filtering out frequencies that do not include the frequency of the paired generating element.

The advantage of this embodiment can be that a plurality of sub-sensor units can be used because each generating and detecting element are linked to each other and, therefore, no overlapping of different sub-sensor units occurs.

In other words, each sub-sensor unit comprises a generating element, e.g. an element which is configured for generating a magnetic field and a detecting element, e.g. an element for detecting a response of a metal object due to the generated magnetic field. Each of the generating elements is configured for generating and/or creating a continuously alternating magnetic field at a predefined frequency. In addition, each of the generating elements is paired with, connected and/or linked to one detecting element. Furthermore, each of the generating elements operates, respectively, runs at a different predefined frequency. In addition, the detecting elements are configured for detecting the continuously alternating magnetic field. Moreover, each of the detecting elements is configured for filtering out, winnow, and/or purifying frequencies that do not include the frequency of the paired generating element.

According to an embodiment, each of the sub-sensor units comprises a generating element and a detecting element. Each of the generating elements is configured for generating electromagnetic pulses in a predefined sequence. In addition, each predefined sequence is configured such that no pulse overlaps in time with another pulse from another generating element. Furthermore, each predefined sequence comprises for each pulse a buffer. Furthermore, each buffer is configured for allowing a measurement of a response of the metal object to the generated pulse. Moreover, each of the detecting elements is configured for measuring the response of the metal object to the generated pulse.

The advantage of this embodiment may be that a measuring with the help of electromagnetic pulses is enabled, since the predefined sequence organizes each of the generating elements such that no overlap occurs and, furthermore, allows the detecting elements to measure a response of the metal objects to the electromagnetic pulses due to the foreseen buffer.

In other words, each of the sub-sensor units comprises a generating element, e.g. an element for generating electromagnetic pulses and a detecting element for measuring a response of the metal objects to the electromagnetic pulses. The generating element is configured for generating and/or creating electromagnetic pulses in a predefined sequence. Each predefined sequence is configured such that no pulse overlaps, coincides and/or clashes in time with another pulse from another generating element. Each predefined sequence comprises for each pulse a buffer and/or a cage. Each buffer is configured for allowing a measurement and/or a detection of a response of the metal object to the generated pulse. Moreover, each of the detecting elements is configured for measuring and/or detecting the response of the metal object to the generated pulse.

According to an embodiment each of the sub-sensor units of the plurality of sub-sensor units comprises a light emitting element, e.g. a LED. Furthermore, the device is configured for activating the light emitting element, when the determined position of the metal object is adjacent to the light emitting element.

This embodiment may have the advantage that the location of the metal object can be identified more easily, since the sub-sensor unit next to the metal object lights up.

A further aspect of the invention is a method for determining a position of a metal object in or at a patient body. The method comprises the steps of:
- generating a magnetic field during a movement of a sensor unit along a trajectory,
- receiving a response signal induced by the generated magnetic field,
- detecting a change of the response signal induced by the generated magnetic field during said movement due to a change of a distance between the sensor unit and a metal object in or at the patient body, and
- determining the position of the metal object based on the detected change of the response signal during said movement.

The advantage of this aspect may be that with the help of the method the detection and localization of metal objects is simplified. This may enable autonomous imaging since metal objects can be localized and removed prior to MRI and X-ray examinations. This can lead to a reduction of preparation effort by supervising personnel. Furthermore, the device may have the advantage that operating personnel of a medical imaging modality can be double-checked, since after the medical personnel supervises the removal of all metal objects, the device can scan the patient again and therefore, the medical personnel may be unburdened.

In other words, the method comprises the step of generating, creating and/or establishing a magnetic field, respectively, a magnetic induction during and/or for the time of a movement of the sensor unit along the trajectory. Furthermore, the method comprises the step of receiving, detecting and/or identifying a response signal, respectively, a reaction induced and/or caused by the generated magnetic field. In addition, the method comprises a step of detecting, identifying and/or measuring a change of the response signal induced by the generated magnetic field during said movement due to a change, a difference and/or a variation of a distance, vector and/or range between the sensor unit and the metal object in the or at the patient body. Moreover, the method comprises the step for determining and/or calculating the position of the metal object based on the detected change of the response signal during said movement.

An embodiment of the invention is the method which further comprises the step of:
- removing the metal object based on the determined position, and/or
- repeating the steps of the method before and hereinafter in order to assure that no metal object is located in or at the patient body.

The advantage of this embodiment may be that with the removing of the metal object, which is indicated by the method, the security can be further improved when a patient, for example, is prepared for an MRI examination.

In other words, the method comprises the step of removing and/or taking away the metal object based on the determined position and/or based on the indicator which is presented by an optical indicator. Furthermore, the method may comprise the step of repeating and/or restarting the steps of the method as described before and hereinafter in order to assure that no metal object is located in or at the patient body.

A further aspect of the invention is a method for autonomous imaging, the method comprising the steps:
- detecting a metal object with a device as described and hereinafter,
- acquiring at least one image with a medical imaging apparatus.

This may have the advantage that with the help of the device autonomous imaging is enabled and therefore supervising personnel can be unburdened. This may also lead to a reduction of costs for medical imaging.

In other words, autonomous imaging comprises the step of detecting and/or identifying a location of a metal object with the device as described before and hereinafter. Furthermore, the method for autonomous imaging comprises the step of acquiring at least one image with the medical imaging apparatus, in particular without the help of medical supervising personnel.

A further aspect of the invention is a computer program element which, when executed, instructs a processor unit to perform the steps of the method as described before and hereinafter.

A further aspect of the invention is a use of the device for determining a position of a metal object in or at a patient body as described before and hereinafter.

All disclosures as described in relation to any aspect of the invention applies equally to all other aspects of the invention. In the following, the examples and embodiments of the invention are described with reference to the Figures.

### BRIEF DESCRIPTION OF THE DRAWINGS

Fig. 1 shows a device according to an embodiment of the present invention.
Fig. 2 shows a device according to an embodiment of the invention.
Fig. 3 shows a device according to an embodiment of the invention.
Fig. 4 shows a device according to an embodiment of the invention.
Fig. 5 shows a device according to an embodiment of the invention.
Fig. 6 shows a flowchart illustrating the steps of a method according to an embodiment of the invention.
Fig. 7 shows a flowchart illustrating the steps of a method according to an embodiment of the invention.
Fig. 8 shows a flowchart illustrating the steps of a method according to an embodiment of the present invention.

### DETAILED DESCRIPTION OF EMBODIMENTS

Fig. 1 shows a device 100 for determining a position of a metal object 200 in or at a patient body 300. The device 100 comprises a sensor unit 102 configured to be moved along a predetermined trajectory 104. Furthermore, the sensor unit 102 is configured for generating a magnetic field 106 during a movement of the sensor unit 102 along a trajectory 104. In addition, the sensor unit 102 is configured for receiving a response signal induced by the generated magnetic field 106. Furthermore, the sensor unit is configured for detecting a change of the response signal induced by the generated magnetic field 106 during said movement due to a change 110 of a distance between the sensor unit 102 and a metal object 200 in the or at the patient body. Moreover, the sensor unit 102 is configured for determining the position of the metal object 200 based on the detected change 110 of the response signal during said movement.

The advantage of this aspect may be that with the help of the sensor unit 102 and the device 100, a presence and a position of a metal object 200 can be detected. This is beneficial for MRI and X-ray examinations where the presence of a metal object 200 is critical, since the metal object 200 may be pulled towards the magnetic field 106 or generates artefacts in the resulting medical image. In addition, the labor can be reduced for preparing a patient for a medical imaging procedure, since the device 100 may enable the patient to remove the metal object 200 by himself, since the device 100 may indicate the position of the metal object 200.

The device 100 comprises a sensor unit 102. The sensor unit is in this example located at the first position 102' and at a second position 102". The sensor unit 102 moves along the predetermined trajectory 104. The sensor unit 102 generates at the first position 102' a magnetic field 106. Furthermore, the sensor unit 102 generates at the second position 102" a second magnetic field 107. The first magnetic field and the second magnetic field 107 both extend up to the metal object 200. A first distance 110 is located between the sensor unit 102 and the first position 102' and the metal object 200. In addition, there is a second distance 112 between the sensor unit 102 at the second position 102" and the metal object 200. The difference between the first distance 110 and the second distance 112 can be indicated as the change 108 of the distance between the first position 102' and the second position 102" of the sensor unit 102. Based on the detected change 108, the location of the metal object 200 located in or at a patient body 300 can be calculated.

Fig. 2 shows a device 100 which comprises a camera 114. The camera 114 is configured for generating an image of the patient body 300. Furthermore, the camera 114 is configured for identifying a contour 302 of the patient body 300. Moreover, the device 100 is configured for adapting the trajectory 104 based on the identified contour 302 of the patient body 300. Furthermore, the device 100 is configured for adapting the trajectory 104 based on the generated image of the camera 114. Moreover, the device 100 comprises an optical indicator 116, e.g. a laser beam. The optical indicator 116 can be, for example, a device for generating an optical indicator so that a laser beam points towards the metal object 200. Therefore, the optical indicator 116 is configured for illuminating the position of the metal object 200 based on the determined position of the metal object 200.

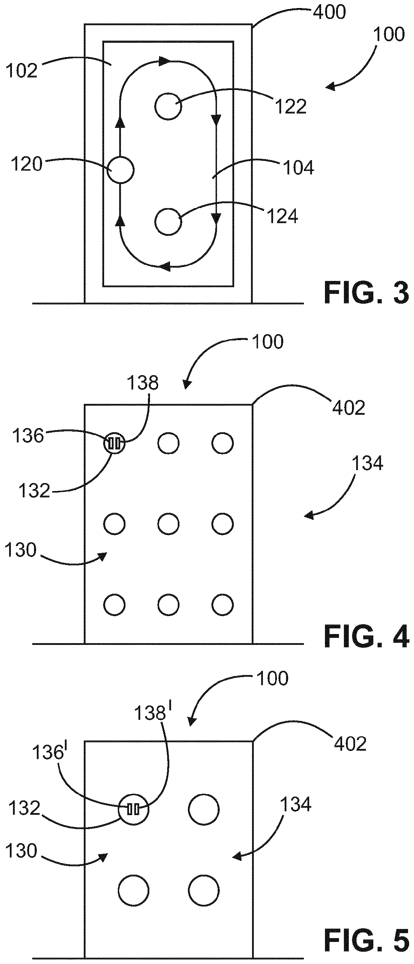

Fig. 3 shows a device 100 which is embodied as a wall element 400. Inside the wall element 400, the sensor unit 102 is located. The sensor unit comprises a first sub-sensor unit 120 which moves along the trajectory 104. In addition, the sensor unit 102 comprises a second sub-sensor unit 122 and a third sub-sensor unit 124. The second sub-sensor unit 122 and the third sub-sensor unit 124 are both located at a fixed position. For example, the second sub-sensor unit 122 and the third sub-sensor unit 124 can detect whether a metal object 200 is present or not. In case a metal object 200 is present, the first sub-sensor unit starts moving and the location of the metal object 200 is determined.

Fig. 4 shows a device 100 which is embodied as a wall 402. The device comprises a sensor unit 102 which comprises a plurality of sub-sensor units 130. An exemplary sub-sensor unit 132 of the plurality of sub-sensor units 130 comprises a generating element 136 and a detecting element 138, wherein the generating element 136 is configured for generating a continuously alternating magnetic field at a predefined frequency. Furthermore, the detecting element 138 is configured for detecting a continuously alternating magnetic field.

Fig. 5 shows a device 100 which is embodied as a wall 402. The device 100 comprises a plurality of sub-sensor units 130. An exemplary sub-sensor unit 132 of the plurality of sub-sensor units 130 each comprises a generating element 136' and a detecting element 138', wherein each of the generating elements 136' is configured for generating electromagnetic pulses in a predefined sequence. Said detecting elements 138' are configured for measuring the response of the metal object to the generated pulse.

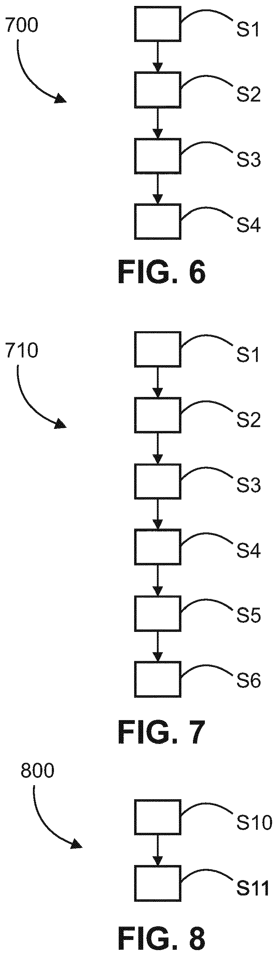

Fig. 6 shows a flowchart of the method 700 which illustrates the steps of generating S1 a magnetic field 106 during a movement of a sensor unit 102 along a trajectory 104. Furthermore, the method 700 comprises the step of receiving S2 a response signal induced by the generated magnetic field 106. In addition, the method 700 comprises the step of detecting S3 a change 108 of the response signal induced by the generated magnetic field during said movement due to a change 108 of a distance between the sensor unit 102 and the metal object 200 in or at the patient body 300. Moreover, the method comprises the step of determining S4 the position of the metal object 200 based on the detected change 108 of the response signal during said movement.

Fig. 7 shows a further method 710 which comprises the steps of generating S1 a magnetic field, receiving S2 a response signal, detecting S3 a change of the response signal, and determining a position of the metal object 200. The further method 710 further comprises the steps of removing S5 the metal object 200 based on the determined position and/or repeating S6 the steps of the method as described before and hereinafter in order to assure that no metal object 200 is located in or at the patient body.

Fig. 8 shows a method 800 for autonomous imaging. The method 800 comprises the step of detecting S10 a metal object 200 with the device 100 as described before and hereinafter. In addition the method 800 comprises the step of acquiring S11 at least one image with a medical imaging apparatus.

The reference symbols used in the Figures are listed in a summary form in the list of reference symbols. In principle, identical parts are provided with the same reference symbol in the Figures.

Where a definite or indefinite article is used when referring to a singular noun, e.g. "a", "an" or "the", this includes a plurality of that noun unless something else is specifically stated. It is to be understood that the term used are interchangeable and under appropriate circumstances, the embodiments of the invention described herein are capable of operating in other sequences than described or illustrated herein.

### LIST OF REFERENCE SIGNS:

- 100: device
- 102: sensor unit
- 102': sensor unit at a first position
- 102": sensor unit at a second position
- 104: predetermined trajectory
- 106: magnetic field at the first position
- 107: magnetic field at the second position
- 108: change
- 110: first distance
- 112: second distance
- 114: camera
- 116: optical indicator
- 120: first sub-sensor unit
- 122: second sub-sensor unit
- 124: third sub-sensor unit
- 130: sub-sensor unit
- 134: pattern
- 136: generating elements
- 136': generating elements
- 138: detecting elements
- 138': detecting elements
- 200: metal object
- 300: patient body
- 302: contour
- 400: wall element
- 402: wall
- 700: method
- 710: further method
- 800: method for autonomous imaging
- S1: generating a magnetic field
- S2: receiving a response signal
- S3: detecting a change of the response signal
- S4: determining the position of the metal object
- S5: removing the metal object
- S5: repeating the method
- S10: detecting a metal object
- S11: acquiring at least one image

## Claims

1. A device (100) for determining a position of a metal object (200) in or at a patient body (300),
the device (100) comprising:
- a sensor unit (102) configured to be moved along a pre-determined trajectory (104),
wherein the sensor unit (102) is configured for generating a magnetic field (106) during a movement of the sensor unit (102) along the trajectory (104),
wherein the sensor unit (102) is configured for receiving a response signal induced by the generated magnetic field (106),
wherein the sensor unit (102) is configured for detecting a change of the response signal induced by the generated magnetic field (106) during said movement due to a change (110) of a distance between the sensor unit (102) and the metal object (200) in or at the patient body (300), and
wherein in the sensor unit (102) is configured for determining the position of the metal object (200) based on the detected change (110) of the response signal during said movement.

2. The device according to claim 1,
wherein the change (110) of the response signal is dependent from the trajectory (104).

3. The device according to any of the preceding claims,
wherein the device (100) comprises a camera (114),
wherein the camera (114) is configured for generating an image of the patient body (300),
wherein the camera (114) is configured for identifying a contour (302) of the patient body (300) based on the generated image, and
wherein the device (100) is configured for adapting the trajectory (104) based on the identified contour (302) of the patient body (300).

4. The device according to any of the preceding claims,
wherein the device (100) comprises an optical indicator (116), e.g. a laser beam, and
wherein the optical indicator (116) is configured for illuminating the position of the metal object (200) based on the determined position of the metal object (200).

5. The device according to any of the preceding claims,
wherein the sensor unit (102) is located inside of a wall element (400), a chair and/or an examination table.

6. The device according to any of the preceding claims,
wherein the sensor unit (102) comprises a first sub-sensor unit (120), which moves along the trajectory (104),
wherein the sensor unit (102) comprises a second sub-sensor unit (122) and a third sub-sensor unit (124), which both are located at a fixed position, and
wherein the device (100) is configured for determining the position of the metal object (200) based on the detected change (108) of the distance during a movement of the first sub-sensor unit (120) along the pre-determined trajectory (104) and the fixed positions of the second sub-sensor unit (122) and the third sub-sensor unit (124).

7. The device according to any of the claims 1 to 5,
wherein the sensor unit (102) comprises a plurality of sub-sensor units (130), wherein each sub-sensor unit (132) of the plurality of sub-sensor units (130) is moveable arranged inside a wall (402),
wherein the device (100) is configured for calculating a shift for each of the sub-sensor units (132) for relocating each of the sub-sensor units (132) of the plurality of sub-sensor units (130) based on a pattern (134), and
wherein the device (100) is configured for moving each sub-sensor unit (132) of the plurality of sub-sensor units (130) based on the calculated shift.

8. The device according to claim 7,
wherein the device (100) is configured for adapting the pattern (134) based on a received probability value, describing where to find metal objects (200) in or at the patient body (300).

9. The device according to claim 8,
wherein the device (100) is configured for changing a sensitivity of each of the sub-sensor units (132) of the plurality of sub-sensor units (132) based on the pattern (134) and the probability.

10. The device according to claim 3 and 7,
wherein the device (100) is configured for adapting the pattern (134) based on the identified contour (302) of the patient body (300).

11. The device according to any of the claims 6 to 10,
wherein each sub-sensor unit (132) comprises a generating element (136) and a detecting element (138),
wherein each of the generating elements (136) is configured for generating a continuously alternating magnetic field at a pre-determined frequency,
wherein each of the generating elements (136) is paired with one detecting element (138),
wherein each of the generating elements (136) operates at a different pre-determined frequency, and
wherein each of the detecting elements (138) is configured for filtering out frequencies that do not include the frequency of the paired generating element (136).

12. The device according to any of the claims 6 to 10,
wherein each of the sub-sensor units (132) each comprises a generating element (136') and a detecting element (138'),
wherein each of the generating elements (136') is configured for generating electro-magnetic pulses in a predefined sequence,
wherein each predefined sequence is configured such that no pulse overlaps in time with another pulse from another generating element (136'),
wherein each predefined sequence comprises for each pulse a buffer, and
wherein each buffer is configured for allowing a measurement of a response of the metal object (200) to the generated pulse,
wherein each of the detecting elements (138') is configured for measuring the response of the metal object (200) to the generated pulse.

13. A method (700) for determining a position of a metal object (200) in or at a patient body (300), the method (700) comprising the steps of:
- generating (S1) a magnetic field (106) during a movement of a sensor unit (102) along a trajectory (104),
- receiving (S2) a response signal induced by the generated magnetic field (106),
- detecting (S3) a change of the response signal induced by the generated magnetic field (106) during said movement due to a change (108) of a distance between the sensor unit (102) and the metal object (200) in or at the patient body (300), and
- determining (S4) the position of the metal object (200) based on the detected change (108) of the response signal during said movement.

14. A method (800) for autonomous imaging, the method (800) comprising the steps:
- detecting (S10) a metal object (200) with a device (100) according to any of the claims 1 to 12,
- acquiring (S11) at least one image with a medical imaging apparatus.

15. A computer program element, which when executed instructs a processor unit to perform the step of the method according to claims 13 and/or claim 14.
